# EUROPEAN PATENT APPLICATION

(11) **EP 4 595 916 A1**
(43) Date of publication of application: **06.08.2025**
(21) Application number: 24155510.1
(22) Date of filing: 02.02.2024
(51) Int. Cl.: A61B 34/20, A61B 5/06, A61B 90/00

(54) **ARRANGEMENT FOR DETERMINING A CURRENT LOCATION OF AN IMPLANTABLE DEVICE DURING IMPLANTATION IN A PATIENT'S BODY**

(71) Applicant: BIOTRONIK SE & Co. KG, 12359 Berlin (DE)
(72) Inventor: Parish, Patrick L., Portland, 97219 (US); Whittington, R. Hollis, Portland, 97202 (US); Reddy, Ravi Kiran Kondama, Portland, 97221 (US); Young, Daniel, Portland, 97202 (US)
(74) Representative: Biotronik Corporate Services SE

(57) **Abstract**

A localizing arrangement (1) for determining a current location of an active implantable device (3) during implantation in a patient's body (5) is described. The features of the localizing arrangement (1) are configured to:
generating a magnetic field (7) wherein the magnetic field (7) is generated using a magnetic field generator (11);
measuring a physical parameter of the generated magnetic field (7) correlating with a distance (d) between the implantable device (3) located within the patient's body and the counterpart device (9), wherein the physical parameter is measured using a magnetic field detector (13); and
determining the current location of the implantable device (3) based on the measured physical parameter of the generated magnetic field (7).

## Description

The present invention relates to a method and an arrangement for determining a current location of an active implantable device such as an implantable leadless pacemaker or an implantable pressure sensor during implantation in a patient's body. The invention is mostly described with respect to an implantable leadless pacemaker but not limited to it. In particular the invention is suitable for an implantable pressure sensor as well.

There are various implantable devices which may serve for monitoring cardiac functions and/or treating cardiac diseases or cardiac malfunctions or pulmonary blood pressure. For example, there are cardiac pacemakers or cardioverter defibrillators.

Implantable devices have been developed with miniaturized dimensions. Such miniaturized devices may be configured such as to be implanted directly into a patient's body, particularly directly into a patient's chest or even directly into a patient's heart. Such implantable devices may be configured to be active devices, i.e. may have their own integrated energy supply, their own integrated logics and/or circuitries, their own electrodes, etc. In other words, such active implantable devices may be implanted for example inside the heart and may then operate autonomously and independently from other devices, particularly independently from devices arranged outside or even distant to the heart. Examples of such miniaturized active cardiac devices are implantable leadless pacemakers (ILP) or implantable cardioverter defibrillators (ICD).

Active implantable devices generally have to be positioned very precisely during an implantation procedure at an intended implantation site. During the implantation procedure, a delivery catheter carrying the implantable device is typically introduced into a vein of the patient and the catheter is then forwarded along the vein towards the intended implantation site at or within the heart. During such procedure, a current location of the implantable device within the patient's body needs to be tracked or monitored precisely.

For such purpose, x-ray fluoroscopy is conventionally applied for navigating the implantable device to the implantation site.

However, such x-ray fluoroscopy generally exposes both, the patient as well as the implanting physician, to x-rays, i.e. to hazardous ionising radiation. Various attempts have been made and approaches have been developed for minimising the amount of x-ray radiation required during the fluoroscopy and/or reducing negative effects of such radiation to the patient and the physician.

There may be a need for an alternative method and arrangement for determining a current location of an active implantable device during implantation in a patient's body. Particularly, there may be a need for a method and arrangement allowing avoiding or minimising negative effects onto the patient and/or a physician during an implantation procedure.

Such needs may be met with the subject-matter of the independent claims. Advantageous embodiments are defined in the dependent claims as well as in the corresponding specification and figures.

According to a first aspect of the present invention, a method for determining a current location of an active implantable device during implantation in a patient's body is proposed. The method comprises at least the following steps, preferably in the indicated order: generating a magnetic field, wherein the magnetic field is generated using a magnetic field generator;
measuring a physical parameter of the generated magnetic field correlating with a distance between the implantable device located within the patient's body and the counterpart device, wherein the physical parameter is measured using a magnetic field detector; and
determining the current location of the implantable device based on the measured physical parameter of the generated magnetic field.

According to a second aspect of the invention, a localizing arrangement for determining a current location of an active implantable device during implantation in a patient's body is described. The localizing arrangement is configured for implementing, i.e. executing and/or controlling, the method according to an embodiment of the first aspect of the invention.

Ideas underlying embodiments of the present invention may be interpreted as being based, inter alia, on the following observations and recognitions.

Briefly summarised in a non-limiting manner, embodiments of the present invention relate to a novel approach for determining the current location of an active implantable device during an implantation procedure in which the implantable device is inserted in a patient's body and is to be located very precisely at a target location forming an implantation site within the patient's body.

As indicated above, such location determination is conventionally implemented using x-ray fluoroscopy.

As x-rays are known to have a negative impact onto living tissue, various approaches have been developed for minimising such impact in order to protect the patient and/or the implanting physician. For example, an x-ray dose emitted during fluoroscopy has been minimised. Furthermore, various protection methods such as an application of x-ray absorbing shields have been implemented, such shields protecting portions of the patient and/or the physician by shielding them from impacting x-rays.

The approach described herein differs from conventional approaches in that, instead of minimising negative effects of ionising radiation during x-ray fluoroscopy, a completely different technique is applied for determining the current location of the active implantable device during an implantation procedure. Particularly, instead of x-ray fluoroscopy, magnetic mapping techniques are applied for localising and guiding the active implantable device during the implantation procedure. Such magnetic mapping techniques rely on generating magnetic fields and then measuring a physical parameter of such magnetic field, wherein the measured physical parameter correlates with a distance between the implantable device and the counterpart device and may therefore be used for finally determining the current location of the implantable device.

In the following, characteristics of embodiments of the present invention will be described in more detail.

The approach described herein may be specifically suitable and/or specifically adapted for precisely determining the current location of an active implantable device in a situation in which such device is implanted in a patient's body.

Such active implantable implantable device may be for example an implantable leadless pacemaker or an implantable pressure sensor. Particularly, the implantable device may be an implantable leadless pacemaker being small enough to be implanted within a cardiac chamber such as a ventricle or an atrium of a heart and being active in a sense that it may generate electrical pulses for supporting a cardiac activity of the patient's heart using its integrated energy source such as a battery, its integrated circuitries and its integrated electrodes for autonomous operation. Alternatively the implantable device may be an implantable pressure sensor being small enough to be implanted within the pulmonary artery and being active in a sense that it may measure the pulmonary blood pressure using its integrated energy source such as a battery, its integrated circuitries and its integrated pressure sensor for autonomous operation.

On the one hand, such active implantable devices are miniaturized to an extend such that they may for example be incorporated into a cardiac chamber within the patient's heart, wherein such miniaturization implies that the devices are difficult to locate and the current location of such device is difficult to monitor. On the other hand, such active implantable devices generally have to be positioned very precisely at an intended implantation site. For example, an electrode of such implantable device may have to be brought into contact with cardiac tissue at a very specific small area within the heart.

Accordingly, it was a long felt prejudice that x-ray fluoroscopy presents the only technique suitable for monitoring the implantable device's current location during the implantation procedure with a sufficient precision.

In overcoming such long felt prejudice, it is proposed herein to use a completely different localisation technique for determining a current position of an implantable device, such localisation technique relying on magnetic mapping instead of x-ray fluoroscopy.

Therein, a magnetic field generator is used for generating a magnetic field in an area including the implantable device located within the patient's body as well as a counterpart device located outside the patient's body. Upon having generated such magnetic field, a magnetic field detector may then be used for measuring a physical parameter of the magnetic field, wherein such physical parameter is selected such that it correlates with a distance between the implantable device and the counterpart. Accordingly, by analysing the measured physical parameter, information about the current location of the implantable device relative to the counterpart device may be obtained. Based on such information, the current location of the implantable device within the patient's body may then be determined.

The magnetic field generator may be arranged at one of the implantable device and the counterpart device. The magnetic field detector may then be arranged at the other one of the implantable device and the counterpart device. In other words, the magnetic field generator may be arranged at the implantable device and the magnetic field detector may be arranged at the counterpart device, or vice versa.

For example, the magnetic field generator or the magnetic field detector may be arranged at the implantable device at a predetermined location of such implantable device, particularly at a location of the implantable device which has to be precisely located during the implantation procedure such as e.g. a tip or an electrode of the implantable device. The other one of the magnetic field generator and the magnetic field detector may then be arranged at the counterpart device at a predetermined location.

Accordingly, knowing the location of the counterpart device arranged external to the patient's body and therefore precisely knowing the predetermined location of the magnetic field generator or magnetic field detector disposed at this counterpart device, as well as determining a relative position of the implantable device in relation to such predetermined external location overall allows to precisely determine the current internal location of the implantable device within the patient's body.

The magnetic field generator may generate the magnetic field such that it covers a volume including the implantable device as well as the counterpart device and has a field strength being sufficiently high all over this volume such as to enable measuring physical parameters with a sufficient precision. Therein, the physical parameter to be measured may show variations which depend on the actual distance between the implantable device and the counterpart device or which actually depend on the actual distance of the magnetic field generator arranged at one of these devices in relation to the magnetic field detector arranged at the other device.

The magnetic field generator may generate the magnetic field with characteristics which vary over time and/or over space. In other words, the magnetic field generated by the magnetic field generator may have different characteristics depending on a location where the magnetic field is measured and/or depending on a point in time when the magnetic field is measured.

For example, the magnetic field generator may comprise a permanent magnet. Such permanent magnet generally generates a stationary magnetic field wherein the magnetic field strength and/or an orientation of the magnetic field depends on a location and, particularly, on a distance of a measuring site in relation to the permanent magnet. The permanent magnet may be provided at low costs and does generally not need any energy supply. Furthermore, the permanent magnet may be small. Accordingly, such permanent magnet may be beneficially provided at the implantable device, e.g. at a tip or an electrode of the implantable device. Alternatively, one or more permanent magnets may be provided at the counterpart device.

Alternatively or additionally, the magnetic field generator may comprise an electromagnet. Such electromagnet may generate a magnetic field upon being supplied with electric energy. Accordingly, a field strength generated by the electromagnet may be varied over time by varying the supplied electric energy. Thus, additional to the characteristics of stationary magnetic fields such as their locally varying field strength and orientation, the electromagnet may generate magnetic fields having field strengths which varies over time. Accordingly, the electromagnet may be used for generating a magnetic field in a pulsed regime in which the magnetic field is for example periodically switched-on and switched-off. Additionally, characteristics of such timely varying magnetic fields may differ locally.

Generally, electromagnets require an energy supply for their operation. Accordingly, in order to save energy in the implantable device, it may be more suitable to include electromagnets in the counterpart device, such counterpart device generally having access to a powerful energy source such as a power grid or a high-capacity battery. However, in principle, an electromagnet may also be included in the active implantable device and may be supplied for example with electric energy from a battery included in such device. Logic included in the implantable device may be configured such that the electromagnet is only operated during the implantation procedure and its operation is stopped after the implantation is completed in order to save electric energy.

The magnetic field detector may be any device being able to detect a magnetic field with regards to its characteristics, particularly with regards to its magnetic field strength and/or orientation. For example, the magnetic field detector may comprise a coil and/or an electrode. The coil and/or electrode may be configured such as to enable measuring characteristics of the magnetic field such as its local variations and/or its variations over time. For example, an electric current induced in the coil due to a varying magnetic field strength may be detected and may form a physical parameter which directly or indirectly depends on the distance between the magnetic field generator and the magnetic field detector.

Additionally or as an alternative, the magnetic field may be generated in a pulsed regime and measuring the physical parameter may comprise measuring pulse transit times. In other words, instead of generating a stationary magnetic field, the magnetic field may be generated with a field strength and/or field orientation varying over time. Such variations over time may be interpreted as magnetic pulses. A time required by such magnetic pulses to transit from the location of the magnetic field generator to the location of the magnetic field detector may be referred to as pulse transit time. As such pulse transit time depends, inter-alia, on the actual distance between the magnetic field generator and the magnetic field detector, measuring such pulse transit times may provide information about such distance.

As a further option, measuring the physical parameter may comprise measuring the physical parameter at at least two different locations. In other words, while it is generally preferred to generate the magnetic field with a single magnetic field generator located at a single magnetic field emission location, it may be beneficial to detect the magnetic field and measure the physical parameter relating to such magnetic field at two, three or more different locations.

For such purpose, two or more magnetic field detectors may be arranged at the counterpart device at different locations and may be used for measuring the physical parameter, which depends on an actual distance relative to the magnetic field generator, at such different locations. Alternatively, a single magnetic field detector may be provided at the counterpart device but may be movable to different locations at the counterpart device such as to sequentially measure the physical parameter at different locations. As a result, distances of such two or more different locations relative to the single magnetic field emission location may be determined. Based on the information about such multiple distances, the location of the magnetic field generator may be determined with an increased precision.

Particularly, the determination of the current location of the implantable implantable device may comprise triangulation based on the measured at least two physical parameters. Expressed differently, upon having measured the physical parameter at two or more different locations, an information about the actual location of the magnetic field generator located for example at the implantable device may be derived with high precision using triangulation techniques. Such triangulation techniques take into account both, the information about the measured physical parameters at the different measurement locations and/or the information about the distance of the different measurement locations relative to the magnetic field emission location derivable thereof, on the one hand, as well as an available information about the different measurement locations themselves, particularly an information about a distance between the measurement locations, on the other hand.

The one or more measurement locations may be arranged at the counterpart device, preferably at or close to a surface of the counterpart device. Generally, the counterpart device may be any device which is able to carry one or more magnetic field detectors or, alternatively, one or more magnetic field generators and which may be arranged outside the patient's body and, preferably, in close neighbourhood to the patient's body during an implantation procedure.

For example, the counterpart device may be a table onto which the patient is to be positioned during the implantation. Such table may be an operation table on which the patient is laying during the implantation operation. One or more magnetic field detectors or magnetic field generators may be integrated into and/or attached to such table. Alternatively or additionally, the counterpart device may be a drape which is adapted to cover the patient during the implantation procedure. Such drape may be a textile component similar to a blanket and may be used for protecting and/or warming the patient during the implantation operation. One or more magnetic field detectors or magnetic field generators may be integrated into and/or attached to such drape. As a further alternative or addition, the counterpart device may be a vest which is adapted to be worn by the patient during the implantation procedure. Such vest may be made with a textile material and may form a piece of clothing which may be worn by the patient as a protection and/or for warming during the implantation procedure. One or more magnetic field detectors or magnetic field generators may be integrated into and/or attached to such vest.

It shall be noted that possible features and advantages of embodiments of the invention are described herein with respect to various embodiments of a localisation method or a localisation arrangement. One skilled in the art will recognize that the features may be suitably transferred from one embodiment to another and features may be modified, adapted, combined and/or replaced, etc. in order to come to further embodiments of the invention.

In the following, advantageous embodiments of the invention will be described with reference to the enclosed drawing. However, neither the drawing nor the description shall be interpreted as limiting the invention.

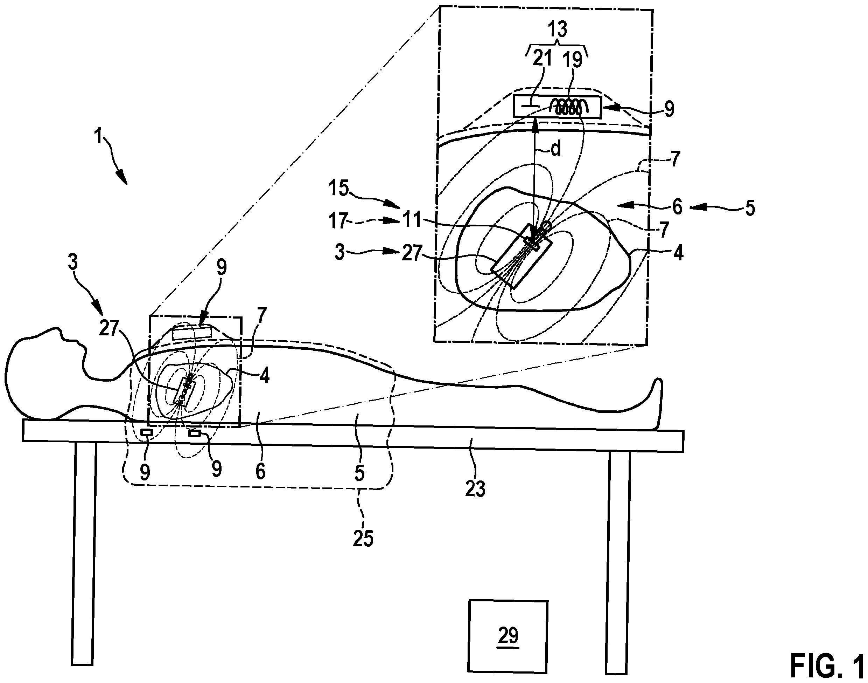

- Fig. 1: shows a localizing arrangement for determining a current location of an active implantable device during implantation in a patient's body in accordance with an embodiment of the present invention.

The figures are only schematic and not to scale. Same reference signs refer to same or similar features.

Fig. 1 shows an embodiment of a localizing arrangement 1. The localizing arrangement 1 is configured for determining a current location of an active implantable implantable device 3 in particular a leadless pacemaker or an implantable pressure sensor during an implantation procedure in a patient's body 5.

During the implantation procedure, the patient's body 5 is arranged on top of a table 23. The active implantable device 3 may be for example a miniaturized implantable leadless pacemaker 27. For being implanted, such pacemaker 27 may be attached to a catheter (not shown) and may then be inserted into the patient's body 5 through a vessel such as a vein towards a chest 6 of the patient. Finally, the pacemaker 27 may arrive at the patient's heart 4, where it shall be fixed to a specific location at cardiac tissue forming a wall of the heart 4.

In order to enable tracking the pacemaker 27 during the implantation procedure and finally correctly positioning the pacemaker 27 at an intended implantation site, the current location of the pacemaker 27 is to be determined continuously or repeatedly. For such purpose, the localising arrangement 1 comprises a magnetic field generator 11 and a magnetic field detector 13. The magnetic field generator 11 generates a magnetic field 7 in a volume spreading throughout the chest 6 of the patient and beyond and including the magnetic field detector 13. The magnetic field detector 13 is configured for measuring a physical parameter of the generated magnetic field 7 correlating with a distance d between the implantable device 3 and a counterpart device 9.

In the given example, the magnetic field generator 11 is arranged at the implantable device 3. Particularly, the magnetic field generator 11 is arranged close to a tip of the pacemaker 27, such tip being intended to be fixed to the cardiac tissue and possibly comprising an electrode transmitting electric signals to such cardiac tissue. The magnetic field generator 11 is formed by a permanent magnet 15. However, such magnetic field generator 11 may alternatively be formed by an electromagnet 17.

Furthermore, in the given example, the magnetic field detector 13 is arranged in or at the counterpart device 9. For example, such counterpart device 9 may be the table 23 on which the patient is to be positioned during the implantation procedure. Alternatively or additionally, such counterpart devices 9 may be a drape 25 to cover the patient during the implantation procedure or a vest (not shown) to be worn by the patient.

In fact, as shown in the figure, a plurality of magnetic field detectors 13 may be included in the localising arrangement 1 and may be comprised in one or more counterpart devices 9 at different locations relative to the implantable device 3.

Each magnetic field detector 13 may comprise a coil 19 and/or an electrode 21 for sensing the magnetic field 7 emitted by the magnetic field generator in 9.

For determining the distance d between the magnetic field generator 11 at the implantable device 3 and the magnetic field detector 13 at the counterpart device 9, a physical parameter of the generated magnetic field 7 correlating with such distance d is measured. For example, a magnetic field strength may be measured with the coil 19 and/or the electrode 21 of the magnetic field detector 13, such magnetic field strength correlating with the position and/or orientation of the magnetic field detector 13 relative to the magnetic field generator 11. More specifically, an impedance may be measured. Alternatively or additionally, in cases where the magnetic field 7 is generated in a pulsed regime, pulse transit times may be measured.

Based on the measured physical parameter of the generated magnetic field 7, a location determining device 29 may then determine the current location of the implantable device 3.

Furthermore, it may generally be beneficial to measure the physical parameter at two or more different locations. For such purpose, multiple magnetic field detectors 13 may be arranged at the different locations. For example, multiple magnetic field detectors 13 may be arranged at the table 23, the drape 25 or the vest (not shown) at various different locations neighbouring to the chest 6 of the patient. In such case, the current location of the implantable device 3 may be determined with high precision using e.g. triangulation techniques based on the various measured physical parameters.

Finally, it should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

### List of Reference Numerals

1 localizing arrangement
3 implantable device
4 heart
5 patient's body
6 chest
7 magnetic field
9 counterpart device
11 magnetic field generator
13 magnetic field detector
15 permanent magnet
17 electromagnet
19 coil
21 electrode
23 table
25 drape
27 pacemaker
29 location determining device
d distance between implantable device and counterpart device

## Claims

1. A method for determining a current location of an active implantable device (3) during implantation in a patient's body (5), the method comprising:
generating a magnetic field (7), implantable device wherein the magnetic field (7) is generated using a magnetic field generator (11);
measuring a physical parameter of the generated magnetic field (7) correlating with a distance (d) between the implantable device (3) located within the patient's body and the counterpart device (9), wherein the physical parameter is measured using a magnetic field detector (13);
determining the current location of the implantable device (3) based on the measured physical parameter of the generated magnetic field (7).

2. The method of claim 1,
wherein the magnetic field generator (11) is arranged at one of the implantable device (3) and the counterpart device (9); and
wherein the magnetic field detector (13) is arranged at the other one of the implantable device (3) and the counterpart device (9).

3. The method of claim 1,
wherein the magnetic field generator (11) comprises a permanent magnet (15).

4. The method of claim 1,
wherein the magnetic field generator (11) comprises an electromagnet (17).

5. The method of claim 1,
wherein the magnetic field detector (13) comprises at least one of a coil (19) and an electrode (21).

6. The method of claim 1,
wherein the magnetic field is generated in a pulsed regime and wherein measuring the physical parameter comprises measuring pulse transit times.

7. The method of claim 1,
wherein measuring the physical parameter comprises measuring the physical parameter at least two different locations.

8. The method of claim 7,
wherein determining the current location of the implantable device comprises triangulation based on the measured at least two physical parameters.

9. The method of claim 1,
wherein the counterpart device (9) is one of
- a table (23) onto which the patient is to be positioned during the implantation,
- a drape (25) to cover the patient during the implantation,
- a vest to be worn by the patient during the implantation.

10. The method of claim 1,
wherein the implantable device (3) is an implantable leadless pacemaker or an implantable pressure sensor (27).

11. A localizing arrangement (1) for determining a current location of an active implantable device (3) during implantation in a patient's body (5), the arrangement (1) being configured for implementing the method of claim 1.

12. The localizing arrangement (1) of claim 11, comprising:
the implantable device (3) to be located within the patient's body (5);
a counterpart device (9) to be located outside the patient's body (5);
a magnetic field generator (11) configured for generating a magnetic field (7), the magnetic field generator (11) being arranged at one of the implantable device (3) and the counterpart device (9);
a magnetic field detector (13) configured for measuring a physical parameter of the generated magnetic field (7) correlating with a distance (d) between the implantable device (3) located within the patient's body and the counterpart device (9), the magnetic field detector (13) being arranged at the other one of the implantable device (3) and the counterpart device (9); and
a location determining device (29) configured for determining the current location of the implantable device (3) based on the measured physical parameter of the generated magnetic field (7).
